# EUROPEAN PATENT SPECIFICATION

(11) **EP 0 907 387 B1**
(45) Date of publication and mention of the grant of the patent: **07.09.2005**
(21) Application number: 96923350.1
(22) Date of filing: 19.06.1996
(51) Int. Cl.: A61N 1/34, A61N 2/00

(54) **MEDICAL ELECTRONIC APPARATUS FOR TREATING HUMAN PAIN**
MEDIZINISCHES ELEKTRONISCHES GERÄT ZUR BEHANDLUNG VON SCHMERZ BEIM MENSCHEN
APPAREIL MEDICAL ELECTRONIQUE POUR AMELIORER LA DOULEUR HUMAINE

(43) Date of publication of application: 14.04.1999
(73) Proprietor: Holcomb, Robert R., Nashville, TN 37203 (US)
(72) Inventor: Holcomb, Robert R., Nashville, TN 37203 (US)
(74) Representative: Fenlon, Christine Lesley
(86) International application number: PCT/US1996/010589
(87) International publication number: WO 1997/002861

(56) References cited:
- US-A- 3 822 708
- US-A- 4 431 002
- US-A- 4 765 310
- US-A- 5 312 321

## Description

### BACKGROUND OF THE INVENTION:

### 1. Field Of The Invention:

This invention relates to the field of medical electronics and more particularly to apparatus for treating human pain by application of an electrical stimulus with the proper current density to the body surface and the response modulated by a magnetic field to allow manipulation of the firing rate of peripheral neurons of the A-fiber and C-fiber nociceptors such that chronic and acute pain may be consistently controlled without discomfort from the stimulation.

### Summary of the Invention

Maurer, et, al., 1994 (Pat # 4,431,002) indicates that it is well known that pain can be alleviated by electrical pulses applied to the surface of the body or to electrodes implanted within the body. His invention revealed a transcutaneous electrical nerve stimulation apparatus in which the stimulus pulses are modulated in both time and intensity in a prescribed manner, the pulse amplitude and width decreasing, while the pulse repetition rate increases and vice versa. The advantage of this arrangement is said to produce a comfortable and pleasant sensation at levels sufficient to produce muscle contraction and stimulation of deep afferent nerves to cause the release of endogenous opiates, such as endorphins, which are thought to suppress pain.

Deyo, et., al., (NEJM) concluded that Transcutaneous Electrical Nerve Stimulation (TENS) in patients with chronic low back pain is no more effective than treatment with Placebo, and TENS adds no apparent benefit to that of exercise alone. It is apparent that such studies are done without the proper application and use of the technology. It is further apparent that technology is needed that is easier to understand and use by the operator.

The reduction of efficacy of a C-fiber input by coactivation of mechanoceptive A-fibers is the principle underlying transcutaneous electrical nerve stimulation (TENS). The mechanism involved is referred to as the "Gate Control Theory of Pain Perception" . TENS involves electrical activation of mechanoceptive fibers. Mechanoceptive A-fibers are activated at lower electrical stimulation intensities than C-fibers, that is, A-fibers have a low threshold. Thus, the mechanoceptive A-fibers can be selectively activated by low intensity electrical stimulation without increasing the firing rate of C-fibers, that is, A-fibers can be selectively activated by low intensity electrical stimulation without increasing the firing rate of C-fibers. As the intensity of stimulation is increased, it is possible to activate both mechanoceptive and nociceptive fibers. Thus, there is a limit to how much stimulation can be applied in order for the current TENS to work. Patients who use TENS devices are fully aware that if they continue to increase the stimulus intensity, they have more pain, rather than less pain. The increasing pain with stimulation is because of C-fiber activation. In some cases, the intensity of stimulation required to achieve pain relief can be reduced simply by repositioning electrodes and reducing the current flux through tissues while still reaching A-fiber threshold: In other cases, it is not possible to achieve pain relief at sufficiently low intensities to selectively activate A-fibers. In these cases, pain may be increased and TENS is said to have failed. In these cases of failure, the information available suggests that TENS failure is largely due to inappropriate electrode placement and insufficient current flow or density at the point of desired stimulation.

Evidence from the literature, clinical observations and isolated neuronal cell preparation data suggest that efficacy of this device is best obtained by high frequency, continuous stimulation with high current density in the area of stimulation. Pacing of A-fibers along with simultaneous suppression of C-fiber firing provides reliable control of pain syndromes. For the efficacy of the invention to be realized, a quadripolar array of positive and negative electrodes are arranged in quadrilateral array such that the positive and negative electrodes are in the proper close proximity to one another such that high current density can be obtained in the area of the nerve fiber to be paced. It is a fiuther object of this invention to suppress the firing rates of C-fibers while increasing the rate of A-fibers. This object is accomplished by placing a Magna Bloc™ device within a stimulating electrode pad housing the electrodes of alternating polarity. This device, as will be demonstrated later, dramatically controls and reduces C-fiber firing. This effect on C-fiber firing is dramatically illustrated in Fig. 7. Volunteer subjects perceived the pain threshold at two (2) times the voltage (which translates to current flow) when the Magna Bloc™ device (MBT) was placed over the stimulating electrode pads as compared to when it was not used (denoted by TH and ST in Figure 7). Through this methodology, normal firing patterns can be sent to the central nervous system, frequency coded, for a sensation of comfort rather than pain.

The device of this invention consists of 4 electrode pads per unit. The electrode pads further consist of 4 electrodes of alternating polarity and consist of 2 positive poles and 2 negative poles. The positive and negative poles of the electrode head are aligned in substantially a single plane and are oriented in a quadrilateral configuration with positive poles oriented diagonally opposite one another and negative poles oriented diagonally opposite one another. Built into each electrode pad is a Magna Bloc™ device (U.S. Patent No. 5,312,321). This device allows maximal A-fiber stimulation without the discomfort of C-fiber pain and muscle contraction. The Magna Bloc™ controls the excitability of neuromuscular units and blocks C-fiber firing.

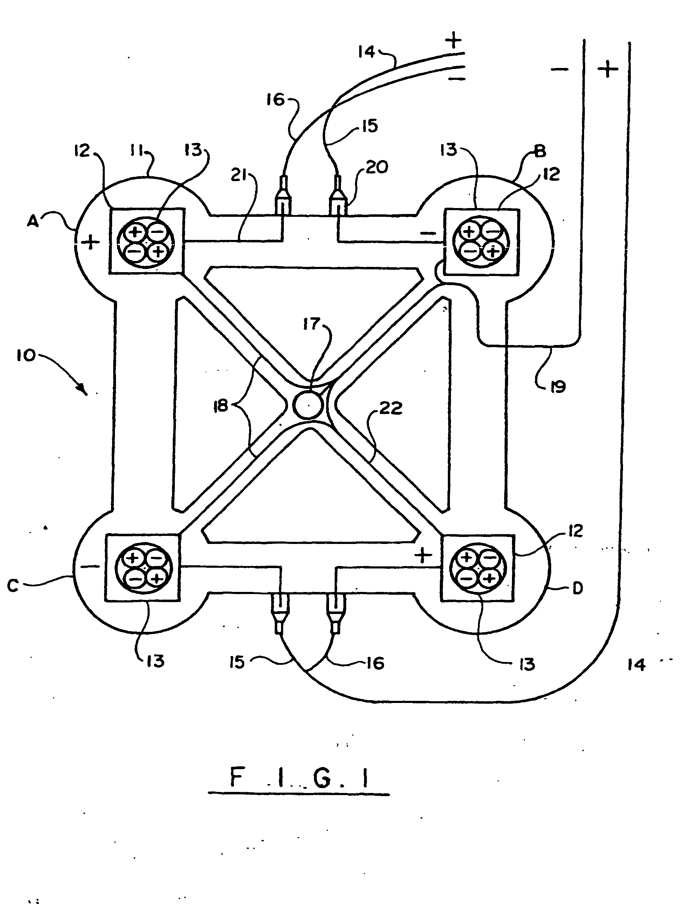

Another object of the invention is to maintain current density sufficient to send A-fiber impulses into the dorsal horn in the area of the innervation of the C-fibers involved in the pain syndrome, in sufficient density to block C-fiber input into the central nervous system. This is accomplished by placing the electrode pads in the correct proximity to each other using the Magna Bloc™ to control C-fiber firing when the intensity is turned up to above usual C-fiber threshold and by placing a current sensor in the midpoint between the 4 electrode pads. This sensor will balance the current density by rotating monitoring of the 4 electrode pads and compensating by changing the input such that current density or current flow in the skin remains constant. This circuit will have a range monitor and alarm system. Current flow will alternate every 2 seconds in the electrode pads shown in Fig. 1, (i.e. from B to A and C to D, C to A and B to D).

It is a further purpose of this invention to have two or more such 4 electrode pads per TENS unit.

Except as noted above, the unit uses standard TENS electronics with the following parameters:
1) the parameters are intensity of output is 0 to 100mA, frequency 0 to 200 Hzt, pulse width 400 microseconds. The device is effective either over the dorsal columns or on afferent nerve bundle as well as over the area of pain sensation. The containment means to hold the 4 electrode pads, 4 Magna Bloc™ devices and current density sensor provides a method of therapeutically placing an alternating electrode DC frequency modulated device on the human body to relieve pain, which has well controlled current density in which C-fiber firing is controlled by a Magna Bloc™ field which generates a flux field gradient in the 'z' axis of 60° to 70°.

This aspect may further provide repetition of these steps for additional containment bodies for attachment to the human body at additional placement positions.

### Brief Description of the Drawings

Fig. 1 is a prospective view of the treatment device electrodes according to one embodiment of the invention;
Fig. 2 is a plan view of the treatment device which powers the electrode of Fig. 1;
Fig. 3 depicts in graphic form field intensity of the magnetic quadripolar portion of the electrodes of the device, as determined by scanning in a systematic parallel place 0.3 cm above the surface of the Magna Bloc™ device;
Fig. 4 depicts useful locations for the placement of the electrodes of the invention; and
Fig. 7 is the change in somatosensory thresholds to electrical stimulus when treated with Magna Bloc™.

### Description of the Preferred Embodiment

Reference will now be made in detail to the presently preferred embodiments of the invention, examples of which are illustrated in the accompanying drawings. Throughout the drawings, like reference characters are used to designate like elements.

The electrode complex of the treatment device of the current invention is schematically illustrated in Fig. 1. Treatment device electrode 10 includes an adhesive means 11 for holding the electrode pads 12 and the Magna Bloc™ Devices 13 in contact with the human body. According to the invention, electrode pad 12 is preferably comprised of 4 electrodes, 2 of which are positive, 2 of which are negative and all of which are electrodes defining opposite diagonal vertices of the quadrilateral shape. Each electrode pad contains a Magna Bloc™ which snaps in position by an aluminum snap.

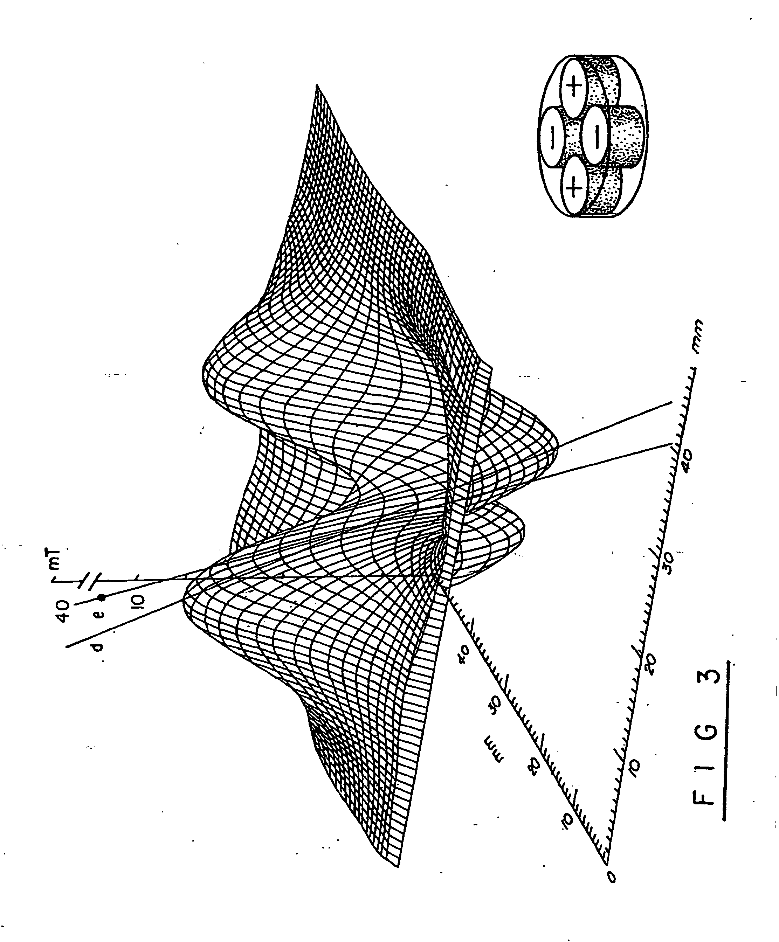

As embodied herein, Magna Bloc™ 13 (magnetic flux generator) comprises four substantially identical magnetic poles held in a plastic containment means that will hold the magnetic bodies in the desired configuration (see U.S. Patent No. 5,312,321) and which Produces a 60° to 70° gradient in the "z" axis (see Fig. 3). The gradient is the slope of the field intensity change over distance.

The embodiment of this invention further contains conducting wires 15 and 16 which connect to electrode wires 21 through connectors 20. The conducting wires 15 and 16 are contained in conducting cable 14. Further embodied in this invention is voltage sensor 37 with electrode connector cables 22 which are ultimately housed in conductor cable 19.

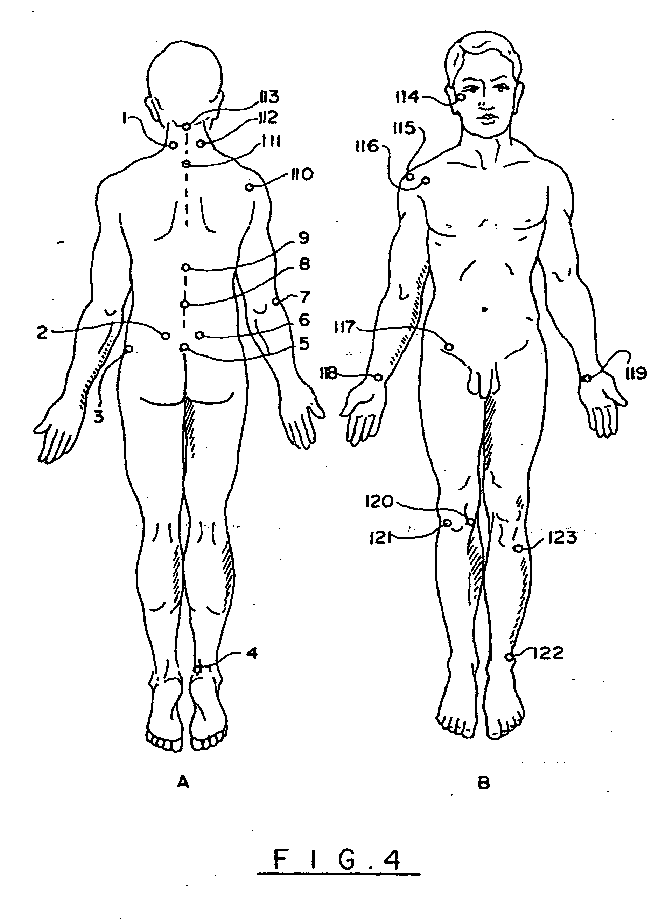

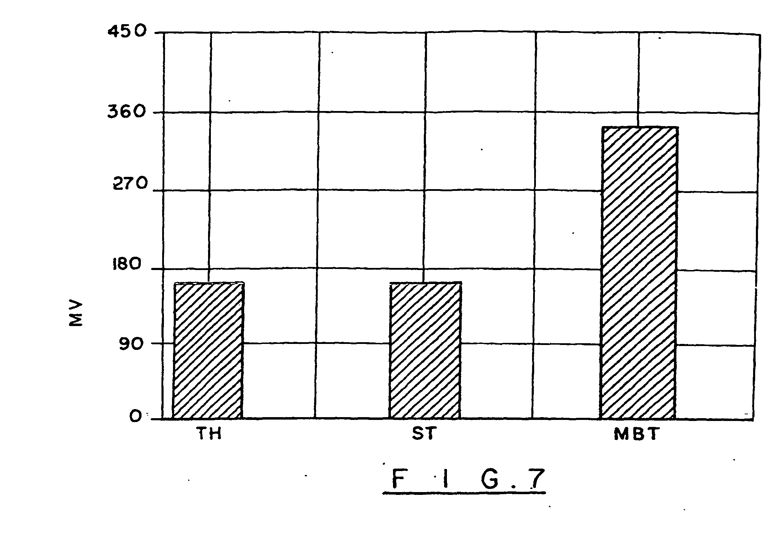

The beneficial effects of this invention are brought about by the ability of the system to maintain a proper current density or flow between the electrodes on a continuous basis in the area of the A-fibers and C-fibers involved in the pain syndrome under treatment. The desired current density is maintained by the electrode pads 12 which are controlled by range monitor (within the housing) and alarm system. The intensity of the current flow will be dictated by a voltage sensor 17. The current flow will alternate every 2 seconds in electrode pads B to A, C to D, C to A and B to D. The density of current flow can be operated at a much higher level than in the classic TENS due to the placement of the Magna Bloc™ device 13 within the electrode pad 12. The Magna Bloc™ 13 completely relieves the discomfort of C-fibrr firing when the C-fiber threshold is exceeded (see Fig. 7). The Magna Bloc™ 13 blocks C-fiber firing, therefore giving a favourable balance to A-fiber/C-fiber ratio and therefore makes this device very effective in relieving pain (see position suggestions for treatment denoted by reference numerals 1 to 9 and 110 to 123 in Fig. 4). For the Magna Bloc™ to control C-fiber firing it must have a field gradient of >45° <90° in the "z" axis.

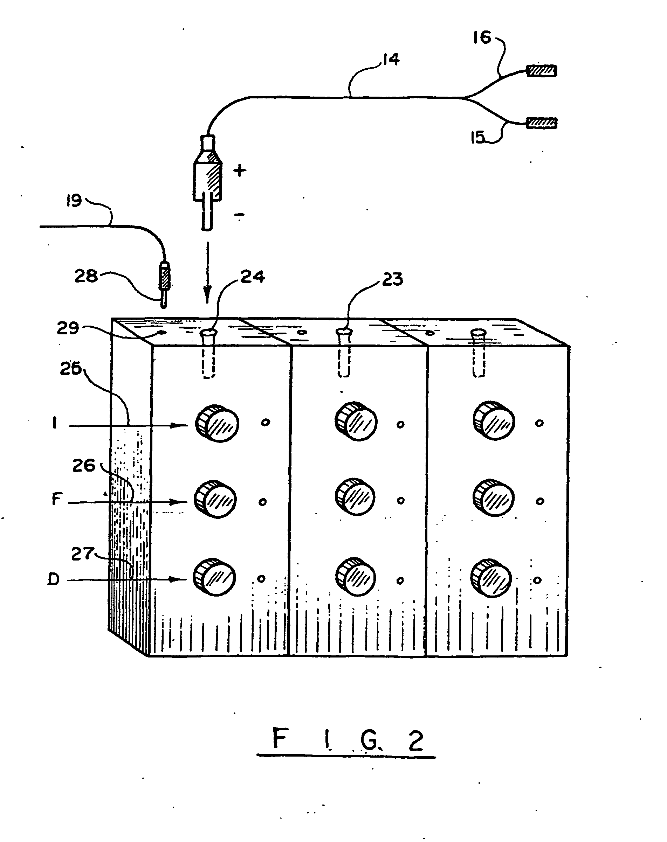

The controlling mechanism of this treatment device as shown in Fig. 2 contains a TENS generator unit 23 which contains the battery source, pulse generator, intensity controls 25, frequency controls 26, duration controls 27, current density modulation polarity switching means, current density modulation cable 19 with male connector 28 and female connector 29. Cable 14 male connector plugs into female connector 24. On-off and alarm light are contained within the cabinet housing.

The data in this document demonstrates that the invention unit will block C-fiber mediated pain and hyperalgesia created by the intradermal injection of capsaicin in human volunteers.

Pain. Pain is a multifactorial perception. The International Association for the Study of Pain defines pain as "unpleasant and emotional experience associated with actual or potential tissue damage."

Pain Pathways and Pain Sensation. The central nervous system integration of pain pathway stimulation takes place in the cerebral cortex. Fig. 4 of the attached drawings presents a schematic representation of the anatomical connections associated with pain perception. The generation of the impulse usually begins in the peripheral nociceptors. The primary afferent neurons are largely A-delta and C-fibers. Pain intensity is related to the firing frequency of the impulses along the pathway. The various structures which have connections along the pain pathway affect the ultimate pain intensity and perception. Very complex circuits affect the pain perception involving both excitatory and inhibitory relationships.

Pain Impulse Generation. The Pain impulse (i.e. volley uf action potentials) may be generated in the nerve ending (i.e. the receptor field) or anywhere along the pain pathway. Any stimulus or injury which results in repeated depolarization of the afferent neuron cell wall will result in a volley of action potentials which are conducted into the central nervous system.

### II. METHODS OF INTERRUPTION OF THE PAIN IMPULSES

A. Electrical Stimulation. Various methods of electrical stimulation have been used in an attempt to control acute and chronic pain. The theories have largely included: 1)The Gate Control Theory and 2)The release of endorphins which block pain transmission. The most widely accepted is the Gate Control Theory. The reduction of efficacy of a C-fiber input by co-activation of mechanoceptive A-fibers thereby increasing the ratio of A-fiber/C-fiber is a principle mechanism. The net result is to increase the frequency of A-fiber firing to C-fiber firing. This results in a net decrease in nociceptor impulses into the central nervous system. Mechanoceptive A-fibers are activated at lower electrical stimulation intensities than C-fibers, that is, A-fibers have a low threshold. Thus, the mechanoceptive A-fibers can be selectively activated by low intensity electrical stimulation without increasing the firing rate of C-fibers. As the intensity of stimulation is increased, it is possible to activate both mechanoceptive and nociceptive fibers. This decreases the ratio of A-fiber to C-fiber firing and, as the stimulus intensity increases, the patient experiences more pain, rather than less, because of C-fiber activation and the decreasing A-fiber/C-fiber firing ratio. In some cases, it is not possible to achieve pain relief at sufficiently low intensities to selectively activate A-fibers. In these cases, pain may be increased with increased stimulus intensity and the TENS is said to have failed.

Nociceptive C-fibers are ordinarily quiescent. However, a tissue-damaging stimulus activates free nerve endings imbedded in the dermis. This transducing step involves the influx of calcium to produce a generator potential. Once the generator potential reaches threshold, action potentials fire and are conducted centrally along C-fibers toward the spinal cord. The net result is that stimulation of C-fibers alters the ratio of A-fiber to C-fiber activation, which in turn increases the firing rate of the T cell and leads to pain perception, according to the Gate Control Theory of pain. The intensity of pain is proportional to the firing rate of the T cell. The invention has been shown to decrease the firing rate of C-fibers both in vitro and in vivo. The alteration in the ratio of A-fiber/C-fiber firing rate results in pain relief.

## Claims

1. A medical electronic apparatus for treating human pain by application of an electrical stimulus, wherein the apparatus comprises:
a) an electrode complex (10) comprising at least one electrode pad (12) that is adapted to be in contact with a human body;
b) said electrode pad (12) comprising positive and negative electrodes defining opposite diagonal vertices of a quadrilateral shape; and
c) power means for supplying power to activate an electrical stimulus to said electrode pad (12);
wherein said electrode pad (12) further comprises at least one quadrapolar magnetic flux generator (13) having four centre charged magnetic poles in alternating polarity suitable for generating a three-dimensional flux field gradient that is greater than 45 ° and less than 90° in the z-axis.

2. The apparatus of claim 1 wherein said electrode complex (10) comprises a plurality of said electrode pad (12).

3. The apparatus of claim 1 or 2 wherein said electrode complex (10) comprises four of said electrode pads (12).

4. The apparatus of claim 2 or 3 further comprising a means for alternating current flow to each electrode pad (12) every two seconds.

5. The apparatus of any one of claims 2 to 4, wherein said electrode pads (12) are approximately 5 centimetres apart.

6. The apparatus of any preceding claim wherein said magnetic flux generator (13) generates a three-dimensional flux field gradient that is greater than 60 ° and less than 70 ° in the z-axis.

7. The apparatus of any preceding claim wherein said magnetic flux generator (13) comprises four circular, centre-charged, neodymium magnetic poles.

8. The apparatus of any preceding claim wherein said electrode complex (10) further comprises a single contiguous unit that includes an adhesive means (11).

9. The apparatus of any preceding claim further comprising a range monitor and alarm system, the range monitor and alarm system receiving sensory input data from a voltage sensor (17) in contact with a surface of said human body and controlling current density supplied to said electrode pad (12) from the sensory input.

10. The apparatus of any preceding claim further comprising a generator unit (23) operatively connected to said electrode complex (10), the generator unit (23) comprising a battery supplying power to said unit, a pulse generator creating a pulsed electrical current through said unit, means for controlling current intensity (25), density, frequency (26) and duration (27) through said electrode complex (10), and means for modulating current density and alternating current direction through said electrode complex (10).

## Patentansprüche

1. Medizinisches elektronisches Gerät zur Schmerzbahandlung beim Menschen durch Anlegen einer elektrischen Stimulation, wobei das Gerät folgendes aufweist:
a) einen Elektrodenkomplex (10), aufweisend mindestens eine Elektrodenlage (12), die dafür ausgelegt ist, mit dem menschlichen Körper in Kontakt zu kommen;
b) wobei die Elektrodenlage (12) positive und negative Elektroden aufweist, welche gegenüberliegende diagonale Eckpunkte einer Viereckform definieren; und
c) Leistungsmittel zum Zuführen von Leistung, um in der Elektrodenlage (12) eine elektrische Stimulierung zu erregen;
wobei die Elektrodenlage (12) mindestens einen quadropolaren magnetischen Flussgenerator (13) aufweist, welcher vier zentral geladene Magnetpole in wechselnder Polarität aufweist, die geeignet sind, einen dreidimensionalen Flussfeldgradienten zu erzeugen, welcher entlang der z-Achse größer als 45° und kleiner als 90° ist.

2. Gerät nach Anspruch 1, wobei der Elektrodenkomplex (10) eine Anzahl der Elektrodenlagen (12) umfasst.

3. Gerät nach Anspruch 1 oder 2, wobei der Elektrodenkomplex (10) vier der Elektrodenlagen (12) umfasst.

4. Gerät nach Anspruch 2 oder 3, weiter aufweisend Mittel zum Wechseln des Stromflusses zu jeder Elektrodenlage (12) alle zwei Sekunden.

5. Gerät nach einem der Ansprüche 2 bis 4, wobei die Elektrodenlagen (12) um etwa 5 cm beabstandet sind.

6. Gerät nach einem der vorstehenden Ansprüche, wobei der magnetische Flussgenerator (13) einen dreidimensionalen Flussfeldgradienten erzeugt, welcher bezüglich der z-Achse größer als 60° und kleiner als 70° ist.

7. Gerät nach einem der vorstehenden Ansprüche, wobei der magnetische Flussgenerator (13) vier kreisförmige, zentral geladene magnetische Neodym-Pole aufweist.

8. Gerät nach einem der vorstehenden Ansprüche, wobei der Elektrodenkomplex (10) eine einzelne zusammenhängende Einheit aufweist, welche ein Haftmittel (11) umfasst.

9. Gerät nach einem der vorstehenden Ansprüche, weiter aufweisend ein Bereichsmonitor- und Alarmsystem, wobei das Bereichsmonitor- und Alarmsystem Sensoreingangsdaten von einem Spannungssensor (17) in Kontakt mit einer Oberfläche des menschlichen Körpers erhält und die Stromdichte, die von dem Sensoreingang der Elektrodenlage (12) zugeführt wird, steuert.

10. Gerät nach einem der vorstehenden Ansprüche, weiter aufweisend eine Generatoreinheit (23), die in Wirkverbindung mit dem Elektrodenkomplex (10) steht, wobei die Generatoreinheit (23) eine Batterie aufweist, welche Leistung an die Einheit abgibt, einen Pulsgenerator, der einen gepulsten elektrischen Strom durch die Einheit erzeugt, Mittel zum Steuern der Stromintensität (25), Stromdichte, Stromfrequenz (26) und der Stromflussdauer (27) durch den Elektrodenkomplex (10) sowie Mittel zum Modulieren der Stromdichte und der Wechselstromrichtung durch den Elektrodenkomplex (10).

## Revendications

1. Dispositif électronique médical destiné à traiter la douleur chez l'homme par l'application d'un stimulus électrique, dans lequel le dispositif comprend :
a) un complexe d'électrodes (10) comprenant au moins un plot d'électrode (12) qui est conçue pour être en contact avec un corps humain,
b) ledit plot d'électrode (12) comprenant des électrodes positives et négatives définissant des sommets de diagonales opposées d'une forme quadrilatérale, et
c) un moyen d'alimentation destiné à fournir du courant pour activer un stimulus électrique vers ledit plot d'électrode (12),
dans lequel ledit plot d'électrode (12) comprend en outre au moins un générateur de flux magnétique quadripolaire (13) comportant quatre pôles magnétiques chargés au centre suivant une polarité alternative, appropriée pour générer un gradient de champ de flux en trois dimensions qui est supérieur à 45° et inférieur à 90° dans l'axe z.

2. Dispositif selon la revendication 1, dans lequel ledit complexe d'électrodes (10) comprend une pluralité de plots d'électrode (12).

3. Dispositif selon la revendication 1 ou 2, dans lequel ledit complexe d'électrodes (10) comprend quatre desdits plots d'électrode (12).

4. Dispositif selon la revendication 2 ou 3, comprenant en outre un moyen destiné à inverser la circulation du courant vers chaque plot d'électrode (12) toutes les deux secondes.

5. Dispositif selon l'une quelconque des revendications 2 à 4, dans lequel lesdits plots (12) sont approximativement espacées de 5 centimètres.

6. Dispositif selon l'une quelconque des revendications précédentes, dans lequel ledit générateur de flux magnétique (13) génère un gradient de champ de flux en trois dimensions qui est supérieur à 60° et inférieur à 70° dans l'axe z.

7. Dispositif selon l'une quelconque des revendications précédentes, dans lequel ledit générateur de flux magnétique (13) est constitué de quatre pôles magnétiques de néodyme chargés au centre, circulaires.

8. Dispositif selon l'une quelconque des revendications précédentes, dans lequel ledit complexe d'électrodes (10) comprend en outre un seul bloc contigu qui comprend un moyen adhésif (11).

9. Dispositif selon l'une quelconque des revendications précédentes, comprenant en outre un système de surveillance de portée et d'alarme, le système de surveillance de portée et d'alarme recevant des données d'entrée sensorielle à partir d'un capteur de tension (17) en contact avec une surface dudit corps humain et commandant la densité de courant fournie audit plot d'électrode (12) à partir de l'entrée sensorielle.

10. Dispositif selon l'une quelconque des revendications précédentes, comprenant en outre un bloc générateur (23) relié fonctionnellement audit complexe d'électrodes (10), le bloc générateur (23) comprenant une batterie fournissant de l'énergie audit bloc, un générateur d'impulsions créant un courant électrique pulsé à travers ledit bloc, un moyen de commande de l'intensité du courant (25), de la densité du courant, de la fréquence du courant (26) et de la durée (27) à travers ledit complexe d'électrodes (10), et un moyen destiné à moduler la densité du courant et à inverser le sens du courant à travers ledit complexe d'électrodes (10).
